(19) Europäisches Patentamt
European Patent Office
Office européen des brevets

(11) **EP 3 162 289 A1**

(12) **EUROPEAN PATENT APPLICATION**

(43) Date of publication:
**03.05.2017 Bulletin 2017/18**

(51) Int Cl.:
**A61B 5/107** (2006.01)    **G06T 7/00** (2017.01)
**A41H 1/02** (2006.01)

(21) Application number: **15425089.8**

(22) Date of filing: **26.10.2015**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA**

(71) Applicants:
• **Akern S.r.L.**
**50065 Pontassieve (FI) (IT)**
• **Talluri, Antonio**
**50012 Bagno a Ripoli (FI) (IT)**

(72) Inventor: **Talluri, Antonio**
**I-50012 Bagno a Ripoli (FI) (IT)**

(74) Representative: **Emmi, Mario**
**Studio Brevetti Turini Srl**
**Viale Matteotti, 25**
**50121 Firenze (FI) (IT)**

(54) **A METHOD FOR ESTIMATING ANTROPOMETRIC AND/OR AUXOLOGIC MEASUREMENTS THROUGH DIGITAL IMAGES**

(57)    The present invention concerns an innovative method for determining one or more anthropometric and/or auxological measurements of a subject.

The method foresees the operations of:
- Acquisition, with a digital device (1), of at least one digital image showing the subject (2);
- Identification of a segment of the image of which the length is known so as to determine a proportion between the number of pixels that compose said segment and its length.

**FIG. 1**

EP 3 162 289 A1

**Description**

Technical field

The present invention refers to the technical field relative to the devices for determining one or more anthropometric parameters of a subject, be it a human or an animal being in general.

In particular, the invention refers to a method that allows to estimate anthropometric and/or auxological measurements, such as measurements in general, asymmetries or scoliosis, through the acquisition of digital images.

Background art

Anthropometry and auxology study the body conformation. The knowledge of body measurements and conformations is essential in the medical field, in the sporting field and in the clothing industry, therefore for the human clinical research and the practice.

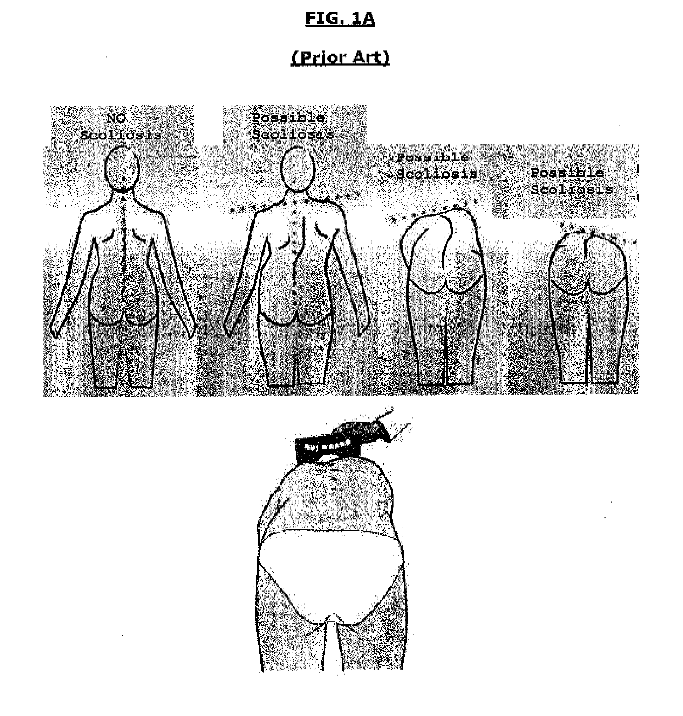

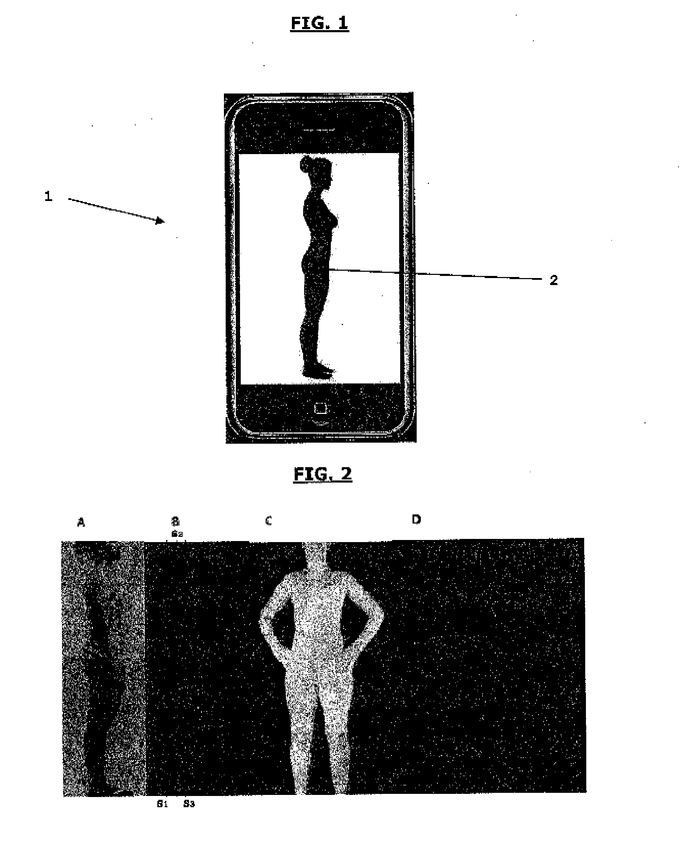

Figure 1A shows postural images to indicate which can be scoliosis conditions. At the current state of the art, always as shown in figure 1A, a scoliosis check needs an idoneous instrument which measures a protrusion or asymmetries of the limbs and behaviors or disturbances of the posture. The subject is positioned upright with the torso bent forward, while the doctor lays on his back a sort of level to control the inclination and thus obtain an indication and quantification of the eventual pathology (Adam's Forward Bend Test).

The same can be said for other anthropometric measurements (for example, the waistline, which is used for instance as a health or cardiovascular risk index) which, to be detected, require not only specific competences, but also the use of specific instruments, such as the traditional tape measure or the anthropometric pliers.

In all cases, therefore, the use of specific instruments and an operator are necessary.

Disclosure of invention

It is therefore the aim of the present invention to provide an innovative method' that solves said technical inconveniences.

In particular, it is the aim of the present invention to provide an innovative method that allows to take anthropometric and/or auxological measurements in a quick and simple way, without the use of already obsolete and traditional instruments.

These and other aims are reached with the method for determining one or more anthropometric and/or auxological measurements of a subject, according with claim 1.

The method foresees the operations of:

- Acquisition, with a digital device (1), of at least one digital image showing the subject (2);
- Identification of a segment of the image of which the length is known, or can be determined, so as to determine a proportion between the number of pixels that compose said segment and its length.

In this way, all said technical inconveniences are easily solved.

In particular, once a segment of the image of which the size is known is determined, for example, the line that identifies the height of the subject is identified and the datum - height h of the subject is inserted, it is at this point immediate the proportion that links a number of pixels of a segment to a length of the segment itself. This allows to determine all the other measurements that are desired, provided that the number of pixels comprised in the section whose measurement wants to be known is known. In this way, measurements of the body can be calculated both for medical investigation purposes and for measurements detection to create clothing.

The applicative fields are therefore significant and the whole is obtained with a quick and simple method.

Further advantages can be deduced from the dependent claims.

Brief description of drawings

Further features and advantages of the present method, according to the invention, will result clearer with the description that follows of one of its embodiments, made to illustrate but not to limit, with reference to the annexed drawings, wherein:

- Figure 1A shows some images of cases of scoliosis and the traditional instrument for its measurement;
- Figure 1 shows a mobile telephony device through which an image is acquired;
- Figure 2 shows an example of acquisition of two images of a subject placed laterally and frontally and with such images processed in order to distinguish background from subject;

- Figure 3 is an example of measurement of two segments of the subject according to the invention;
- Figure 4 and 5 are an example of measurement of the waistline according to the invention;
- Figure 6 is an example of possible variant of the invention;
- Figure 7 is an example of image acquisition for a check of possible scoliosis;
- Figure 8 shows possible examples of acquisition of the idoneous measurements to define the size of a person;
- Figure 9 shows an alternative that allows the counting of the pixels comprised between two horizontal straight lines.

Description of some preferred embodiments

In figure 1, according to the present invention, a mobile telephony device 1 is represented, for example of the I-Phone or Smart-Phone type or a portable device provided with a photo-camera and Wi-Fi connection such as a tablet, a portable PC and the like.

Such devices, mainly those of mobile telephony (of relatively recent diffusion) have become of common use and are always substituting more and more the old mobile phones with limited functions.

In fact, they are provided with much more powerful processors, therefore they result to be small portable PC capable of making many more operations with respect to a classic mobile phone. In particular, in addition to allowing to make normal calls and to send messages, they are provided with Internet connection and, in a quick and easy way, allow to surf the web. Applications (called in technical jargon just "App") are always more diffused, many of which are downloadable also free of charge, and which allow, once downloaded, to do multiple operations that are precisely implemented through the processor of the telephone in which they have been installed.

There exist applications in all fields that range from entertainment and work to other uses in general.

The present invention, which is described in detail below, can for example be implemented in the form of an "App" compatible with the diffused operative systems (Apple, Android, Windows, etc.), downloadable on the mobile telephony device or tablet, and which will be described in detail below.

Nevertheless, a software with the same functionalities for tablets or pc, devices that are often provided with a photo-camera, cannot be excluded.

Alternatively, it is naturally possible to foresee a mobile telephony device that includes a SIM or a processor already programmed for functioning in accordance with the present invention (therefore without the need for downloading a specific application).

In a further alternative, as clarified below, a part of the operation can be implemented by the mobile telephony device or by the device connectable to the net which subsequently sends certain information to a dedicated system put online or in a still more recent system called "cloud" that executes the rest of the operations to give a result.

Having said that, figure 1 represents a mobile telephony device 1 (or, as said, a mobile device) that acquires a digital image through the own photo-camera, an image that shows a profile of a subject 2.

The subject has preferably to wear a bathing suit or just underwear, rendering the profile, and therefore the shape of the own body, well visible.

In such a manner, the whole body will be characterizable by an image with pixels of colours very similar one to the other.

The background for the acquisition of the image has to be preferably of a homogeneous colour idoneous to create a contrast with the colour of the subject, for example white and still more preferably not reflecting, so as to obtain a net difference of colour between the acquired image and the background and possibly the last one not creating reflections.

A simple wall of a house, generally white, can be used, for example.

An eventual interpolation, easily realizable as clarified below, can complete the discontinuity created by the eventual section of the body covered with bathing suits or underwear.

As clarified below, the images, according to the measurement to be taken, can be one or more than one, for example in lateral position with the arms that are along the body and/or frontal, with the limbs abducted or with the subject inclined forward.

The software, implemented in the mobile device, for example through a specific "App", requires the insertion of some data, among which the height (h) of the subject.

The data (as said, height, but also other eventual ones such as age, sex, level of physical activity, etc.) can be requested and be inserted before or after the acquisition of the image.

Therefore, the user acquires a series of images, one or more than one, and when he starts the application the system requests to load the image/s through which to make the desired measurements. The programme then requests the insertion of the data, such as the height of the subject, to then process the whole as described below.

When the image is inserted, and with reference for example to figure 1, the software implements a phase of blocking out, or contrast, between the subject acquired and the background, in order to distinguish well the silhouette of the subject from all that is external to it in the acquired image, therefore creating a good contrast and consequently a good profile.

In order to do this, a comparison of image recognition, of threshold of colouring and luminosity between the pixels is realized, distinguishing and defining the image of the subject with respect to the background. In particular, the user, through for example the Touch Screen system already of common use in such mobile devices, is guided to touch the internal area of the silhouette with a finger in such a way that the software acquires the logical information of luminosity of the pixels belonging to the area touched. In this way, the software acquires the information that all the pixels of a colour equal or similar to those of the area touched belong to the silhouette of the subject. At this point, the value of luminosity of the pixels belonging to the "shown" subject is noted, and therefore the fact that all the pixels having such a luminous intensity will belong to the figure to be examined is also known.

Subsequently, the software implements a comparison of luminosity by scanning all the pixels of the image, starting for example from the superior angle towards the inferior angle and moving in a line-line (or column-column) manner. All the pixels that are below or above said threshold of luminosity are kept unaltered, while the others are for example modified to a high contrast colour with respect to the background, for example reds (therefore white background and red profile image). Alternatively, both (background and image) can be modified precisely to create a still greater contrast (for example, white subject and black background). In that case, all the pixels that are below or above said threshold of luminosity derived from the area touched are brought to a certain value of luminosity, while the others are brought to another value of luminosity in contrast with the preceding one. In this way, an image is created made of two colours in strong contrast with a significant cleaning from "noise", that is the lines result to be well defined.

Such a technique, for example through the use of the touch screen, allows to interpolate also the areas covered by clothes, for example the underwear. By simply touching such a specific area the software interprets this information showing the pixels belonging to such an area to the colouring used for the subject 2, or by allotting to the highlighted pixels the same value of the pixels that the skin shows.

An efficient alternative is that of using the number assigned at international level to the RGB pixels (Red-Green-Blue) whose mathematical summation and manipulation allows to identify significant variations to discriminate easily the silhouette of the body from the background, having the two surfaces colours of different RGB composition. The acronym RGB indicates how said three colours, "red, yellow and blue", which are the three fundamental colours of the pixels from which all the other colourings are created. It is known that a background that is white, for example, will have three RGB components of a pra-determined value (R=255;G-255;B-255), while the skin has a sum of the three components of a second predetermined average value (example, Caucasian people have average RGB values of, respectively, R:239, G:208, B; 207). Being such summations known *a priori*, then it is easy to do an analysis of threshold to distinguish background from subject, in this case without the need that the user touches the silhouette shown.

At this point, a net contrast of colour has been created between the pixels of the "shown subject" image and those of the background.

Starting from a background condition with deeply different pixels as regards colouring with respect to the shown shape, the calculation of the anthropometric measurements can be done, as described below.

Figure 2 shows, for example, a "blocking out" of two images, a frontal and a lateral one, with the cases (A-B-C-D).

Once the image has been blocked out, a measurement of the shown subject known a priori, for example the height h, it is possible to count the number of pixels relative to a line indicative of such a measurement of the subject, for example the height, and obtain by proportion other measurements of other sections.

The use of the height datum is preferred since it is a certain reference datum that each subject knows and can insert.

Once the user has inserted, as requested, the height datum, the software proceeds with identifying the line that in the image of the subject identifies the height measurement.

For instance, the software, as shown schematically in figure 2 - Case B, can scan the image column by column and check which is the column with the greatest number of pixels belonging to the colouring of the pixels of the subject. The column with the greatest number of pixels of colouring of the subject is assumed as the column corresponding to the height of the subject. Figure 2-B indicates, as a way of example, some red lines (S1, S2, S3) that represent the scanning that is done by the software on the image. In the case of measurements of the height h a lateral image of the subject is preferably analyzed since, if placed frontally and with divaricated legs, the measurement could be distorted.

The images of figure 2 show part of the head cut out only for privacy matters but it is evident that, in the case of entire images, the scanning column - column will determine columns with different numbers of pixels as we move from a side of the image of the subject towards the opposite side given the presence of the head and, generally, the height h corresponds to the column of pixels that passes through the center of the head.

Once the column of pixels corresponding to the height has been identified, a simple proportion is obtained that allows to obtain all the other measurements.

If, for instance, the subject is 170cm high, the software, once the column (S1, S2, S3) of pixels corresponding to the height (for example, the column S2) has been identified, can easily count the pixels contained in such a column and create a proportion between such a number of pixels and the height inserted.

If, just as a way of example, the straight line S2 of figure 2 identifies the height of the subject and contains 1.000 pixels, this means that to a height of 170cm of the subject corresponds a number of pixels of 1.000. Such a datum allow, as clarified below, to obtain all the other measurements that are desired.

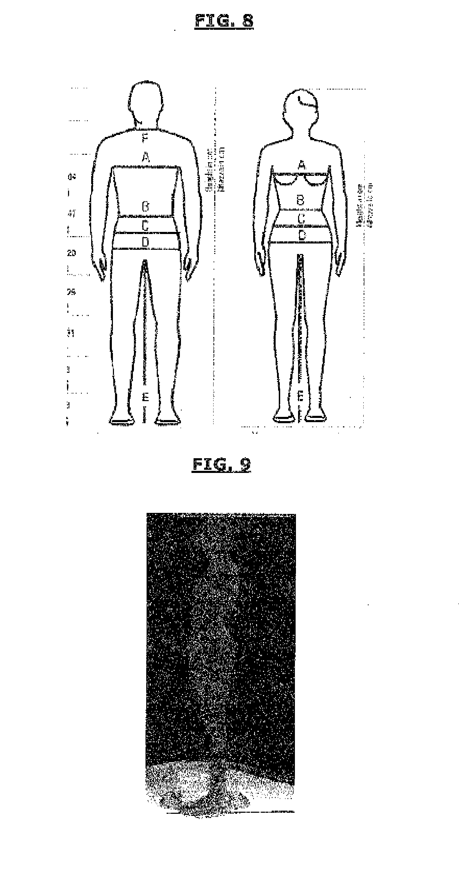

Alternatively, as schematized in figure 9, a system of dragging of two horizontal lines traced apically to the head and below the feet permits also to determine the pixels-height relation since it is enough to identify the vertical coordinates on the screen of the two bars.

The vertical coordinates of the two straight lines are easily identifiable. It is in fact known that each screen has a certain resolution defined by the number of pixels contained in horizontal line and in vertical line. Therefore, starting from a zero of origin that can be, for example, an angle of the image, the number of pixels in vertical and in horizontal is known. When the software traces a line, for example horizontal, this is traced in a position known *a priori* and defined by default, for example precisely in correspondence of the angle of origin of the image or in the midpoint or in other known positions. Such a positioning is identified as initial reference position of which the number of pixels with respect to the origin is known (an essential datum precisely to trace the line) and, therefore, any further movement of the straight line allows to obtain the number of pixels with respect to the origin.

At this point, when the user translates with the touch screen the straight line up or down, the software counts the number of pixels of the dragging and, known the initial position of the beginning, determines the overall number of pixels comprised between straight line and origin or between two or more parallel straight lines.

In the case of example of figure 9 with two horizontal straight lines, one of such straight lines, once traced by the software in a known initial position, is moved onto the head of the subject, and the coordinate or the number of line of the pixels with respect to the origin is therefore known. The other one is moved in correspondence of the feet and likewise the coordinate or the number of line of the pixels with respect to the origin is known. At this point, by difference, the number of pixels comprised between the straight lines is known.

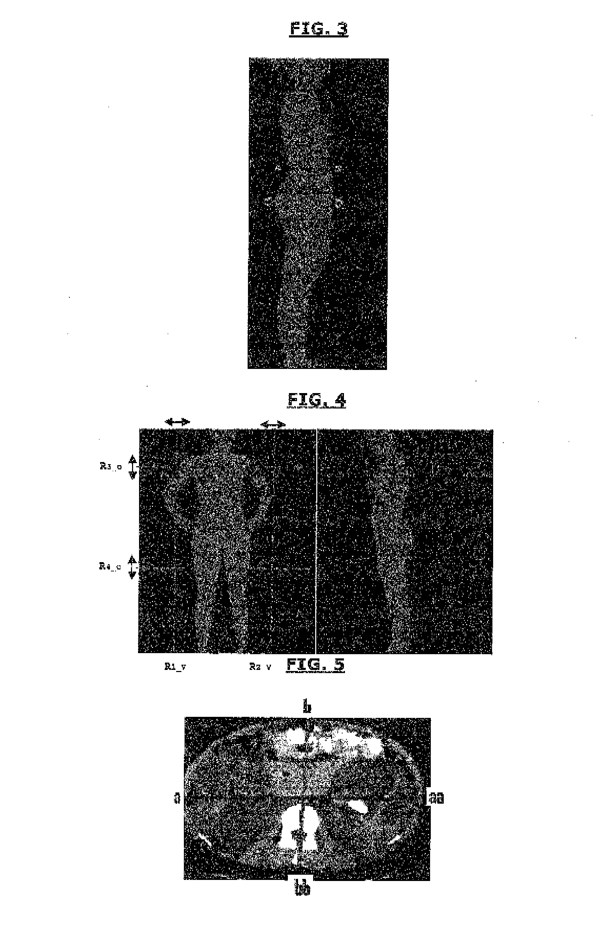

Having said that, once such a proportion has been determined between a segment identified in the image and its size, with reference to figure 3, is it now possible to know immediately any further measurement identified in the image, for example the measurement of the segment A-B and/or of the segment C-D that intersect the torso of the subject. If the segment of horizontal straight line of figure 3 (the segment A-B) contains 300 pixels, then by a very simple proportion the measurement in centimeters of the horizontal straight line A-B can be obtained, that is

$$(A-B)\_Horiz.\ straight\ line = (300/1.000) \times 170\ (cm)$$

Once such measurements are known, multiple anthropometric measurements can be easily obtained. The same can be said for the C-D straight line.

For example, figure 4 and figure 5 show the case of acquisition of two images, that is a frontal and a lateral one. By generating, for instance, a horizontal line at the level of the navel, leaving as it is what has been previously said, the lengths of the axes (a-aa; b-bb) relative to the sides are determined immediately, as shown in figure 5, at the height of the navel.

At this point, to know the waist circumference in correspondence of the height of the navel, known the axes, becomes immediate. This is because any section of the human body, including the section of the human torso, is near an ellipse of which it is possible to determine, as described, major axis (a-aa) and minor axis (b-bb).

As it is well known, the circumference of the ellipse is calculated as:

$$P = 2 \times Pi \quad \times\ square\ root\ of\ (a^2 \times b^2 / 2)$$

At this point, not only is it possible to determine the circumference-waist of the subject, but we can also estimate the volumes present between two sections of circumference (for example, the volume comprised between the sections A-B and C-D of figure 3).

Having said that, the software allows the user to trace horizontal lines and vertical lines (one or more than one) in order to detect the desired measurements (previous acquisition of image, insertion of the own height and determination of the proportion between the height segment and relative number of pixels).

For instance, automatically the two horizontal straight lines A-B and C-D of figure 3 can be traced and can be translated between them to make them come close or move apart, as the traceability of a single line would also be possible.

Preferably, the system allows to trace a box made of two vertical straight lines and two horizontal straight lines and such a box, through the touch screen, can be moved by the user by making the straight lines come close and move apart between them.

Figure 4 in fact shows, for that purpose, an example of such a box with the lines R1_v, R2_v, R3_o and R4_o and the double direction of the arrow is shown through which they can be moved between them.

The single straight line R3_o can, for example, be used translating it in the desired position (for example, height of the navel) to measure such a length (the section a-aa of figure 5).

It is therefore evident that the possibilities of body measurement are multiple, allowing the system described even to approximate a length of the nose on a profile image, by simply using two vertical straight lines appropriately placed between them and obtaining the reciprocal distance thereof.

For each straight line that is traced, and eventually translated in a specific position, the software is capable of counting the pixels comprised between a straight line and the other one, as described above, and from here to obtain the distance between the straight lines in measurement through the known proportion determined above.

In the same manner, the software is capable of counting the pixels of the segment of straight line contained in the section of intersection with the profile of the subject. In particular, in correspondence of a positioning of the straight line, the pixels belonging to the subject are counted by that line or column (according to horizontal or vertical straight line) and said pixels are therefore identified by the specific colouring of the pixels of the subject (the height of the straight line as said above is in fact known). At this point, always with the same known proportion, the measurement is obtained.

It is at this point evident that the present method allows also to do checks of scoliosis or deformities on the subject.

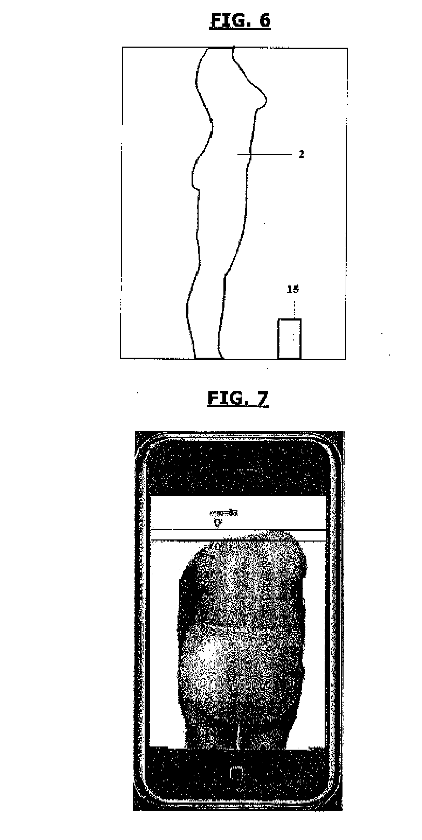

As for example shown in figure 7, leaving as it is what has been previously said, it is possible on the image to trace the two horizontal lines and translate them in the position shown in figure 7 in such a way that the software automatically extrapolates the distance between the two straight lines. Such a distance is indicative of a scoliosis.

From what has been described above, therefore, it can be deduced that it is not necessary anymore to use special and specific medical equipment, for example "hardware", but instead a software" is enough.

The doctor himself, or anyone who desires it, for example by simple downloading a specific application to his own mobile phone, is capable of obtaining an anthropometric and/or auxometrical measurement in a quick and easy way.

As shown in figure 8, the present invention allows also to calculate multiple horizontal and/or vertical lengths, therefore allowing to obtain many useful measures in the clothing field (textile), for example to create custom-made clothes. For instance, multiple horizontal lines can be traced and the pixels of the subject at the height of the straight lines traced can be counted, to then obtain the measurements.

The operation of creation of contrast in the image between subject and background is not essential if *di per sé* the image is acquired with a homogeneous background that creates enough contrast with respect to the homogeneous colour of the subject. Nevertheless, the contour optimizes the contrast and eliminates the "noise", therefore allowing a more precise measurement.

Figure 6 shows a further variant of the invention.

Leaving as it is what has been described, in fact the proportion between a line of the image and one of its known measurements does not have to be necessarily considered by the introduction of a height of the subject and the identification of the vertical line of pixels that identifies such a height. In fact, in such a variant, as shown in figure 6, a reference object can be used of known, size. Naturally, the software has to be arranged for that purpose. The image of the subject is therefore shown together with the reference object and a contrast is created between background, object 15 and subject 2 exactly as described. The object 15 can be any object of know size, such as a can of Coca-Cola, provided that in the programme is inserted the insertion of the datum "height of the known reference object" (datum preferably inserted already by default and therefore not requested to the user).

At this point, the software, with the usual analysis of the vertical columns, identifies the column relative to the can (it will be that with the minimum number of pixels with a colouring different from the background) and associates to such a number of pixels the quota of height known.

**Claims**

1. A method for determining one or more anthropometric and/or auxological measurements of a subject, the method foreseeing the operations of:

   - Acquisition, with a digital device (1), of at least one digital image showing the subject (2);
   - Identification of a segment of the digital image of which the length is known so as to determine a proportion between the number of pixels of said segment and its length.

2. A method, as per claim 1, wherein a phase of contrast of the subject with respect to the background is foreseen.

3. A method, as per claim 2, wherein said phase of contrast foresees a colouring of the pixels that compose the subject (2) with a color in contrast with respect to the pixels that compose the background.

4. A method, as per claim 2 or 3, wherein said digital device (1) is provided with a screen of the "touch screen" type, said phase of contract foreseeing the operation of touching through the screen an area of the image in such a way that a scan is made of the entire image, putting all the pixels with a colouring different from those belonging to the area touched in a tonality of colour different with respect to that of the pixels having a colouring similar or equal to those of the area touched.

5. A method, as per one or more of the preceding claims, wherein said known length is the height (h) of the subject (2) shown in the image.

6. A method, as per one or more of the preceding claims, wherein the identification of the segment associated to the height (h) of the subject foresees a phase of scanning of all the vertical columns of pixels that compose the image in such a way as to identify the column of pixels that compose the subject having the greatest number of pixels.

7. A method, as per one or more of the preceding claims, wherein the identification of the segment associated to the height (h) of the subject foresees the tracing of two horizontal straight lines and the translation thereof, for example through the touch screen type of screen or a pointing system such as the mouse or pen, in correspondence of the apex of the head and of the feet,

8. A method, as per one or more of the preceding claims, wherein a phase of tracing is subsequently foreseen of at least one horizontal line (R3_0, R4_o) and/or of at least one vertical line (R1_v, R2_v) in such a way that said lines intersect the image of the subject to derive the measurement of the segment (A-B, C-D) intersected with the profile of the subject (2) and/or the distance between said straight lines.

9. A method, as per one or more of the preceding claims, wherein a phase of tracing is subsequently foreseen of two horizontal lines (R3_0, R4_o) and/or of two vertical lines (R1_v, R2_v) translatable between them, for example through the "touch screen" type of screen or pointing systems such as the mouse or pen, and so that the reciprocal distance can be determined between said straight lines and/or the measurement of the segments determined by the intersection of said straight lines with the subject.

10. A method, as per one or more of the preceding claims, wherein the digital device is a mobile telephony device, for example an 1-Phone.

11. A method, as per one or more of the preceding claims, wherein the digital device is a Tablet or a portable PC provided with a digital photography system.

12. A mobile telephony device **characterized in that** it is programmed in such a way as to function in accordance with the method according to one or more of the preceding claims from 1 to 11.

13. An application for a mobile telephony device **characterized in that** it functions in accordance with one or more of the preceding claims from 1 to 11.

14. A software application for a personal computer (PC) or tablet **characterized in that** it functions in accordance with one or more of the preceding claims from 1 to 13.

## FIG. 1A

## (Prior Art)

## FIG. 1

## FIG. 2

## FIG. 3

## FIG. 4

## FIG. 5

## FIG. 6

## FIG. 7

FIG. 8

FIG. 9

Europäisches
Patentamt

European
Patent Office

Office européen
des brevets

# EUROPEAN SEARCH REPORT

Application Number

EP 15 42 5089

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2014/270540 A1 (SPECTOR DAVID [US] ET AL) 18 September 2014 (2014-09-18)<br>* paragraph [0178] - paragraph [0187] *<br>* paragraph [0075] - paragraph [0105] *<br>* paragraph [0045] - paragraph [0048] *<br>* figures 1,10-21,24 * | 1-4,9-14 | INV.<br>A61B5/107<br>G06T7/00<br>A41H1/02 |
| X | US 2015/223730 A1 (FERRANTELLI JOSEPH RALPH [US]) 13 August 2015 (2015-08-13)<br>* paragraph [0050] *<br>* paragraph [0054] - paragraph [0065] *<br>* paragraph [0114] - paragraph [0121] *<br>* figures 1-9 * | 1,5,7,<br>10-14 | |
| X | GB 2 449 648 A (SONY COMP ENTERTAINMENT EUROPE [GB]) 3 December 2008 (2008-12-03)<br>* page 19, line 8 - line 34 *<br>* claims 1,2,23,24 *<br>* figures 4-6,8 * | 1,5,6 | |
| X<br>A | US 2009/062693 A1 (WOOLFSON DAVID [IE] ET AL) 5 March 2009 (2009-03-05)<br>* paragraph [0047] - paragraph [0059] *<br>* figures * | 1,8<br>9 | TECHNICAL FIELDS SEARCHED (IPC)<br>A41H<br>A43D<br>A61B<br>G06T |
| X<br>A | US 2010/246898 A1 (IZUMI KENJI [JP]) 30 September 2010 (2010-09-30)<br>* paragraph [0053] - paragraph [0059] *<br>* figure 21 * | 1-4<br>6 | |

The present search report has been drawn up for all claims

| Place of search | Date of completion of the search | Examiner |
|---|---|---|
| The Hague | 22 April 2016 | Görlach, Tobias |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

& : member of the same patent family, corresponding
document

EPO FORM 1503 03.82 (P04C01)

## ANNEX TO THE EUROPEAN SEARCH REPORT
## ON EUROPEAN PATENT APPLICATION NO.

EP 15 42 5089

This annex lists the patent family members relating to the patent documents cited in the above-mentioned European search report.
The members are as contained in the European Patent Office EDP file on
The European Patent Office is in no way liable for these particulars which are merely given for the purpose of information.

22-04-2016

| Patent document cited in search report | | Publication date | Patent family member(s) | | Publication date |
|---|---|---|---|---|---|
| US 2014270540 | A1 | 18-09-2014 | US 2014270540 A1<br>WO 2014159726 A1 | | 18-09-2014<br>02-10-2014 |
| US 2015223730 | A1 | 13-08-2015 | NONE | | |
| GB 2449648 | A | 03-12-2008 | GB 2449648 A<br>WO 2008145952 A1 | | 03-12-2008<br>04-12-2008 |
| US 2009062693 | A1 | 05-03-2009 | US 2009062693 A1<br>US 2011009776 A1 | | 05-03-2009<br>13-01-2011 |
| US 2010246898 | A1 | 30-09-2010 | CA 2707912 A1<br>JP 5124886 B2<br>TW 200938276 A<br>US 2010246898 A1<br>WO 2009072435 A1 | | 11-06-2009<br>23-01-2013<br>16-09-2009<br>30-09-2010<br>11-06-2009 |

EPO FORM P0459

For more details about this annex : see Official Journal of the European Patent Office, No. 12/82